# EUROPEAN PATENT APPLICATION

(11) **EP 3 133 165 A1**
(43) Date of publication of application: **22.02.2017**
(21) Application number: 15181188.2
(22) Date of filing: 17.08.2015
(51) Int. Cl.: C12Q 1/68

(54) **METHODS FOR PERSONALIZING PATIENT CANCER THERAPY WITH ANTI ANGIOGENIC COMPOUNDS**

(71) Applicant: F. Hoffmann-La Roche AG, 4070 Basel (CH)
(72) Inventor: The designation of the inventor has not yet been filed
(74) Representative: Beyermann, Jochen Carl

(57) **Abstract**

The present application discloses a method to predict responsiveness of a patient, with cancer, to treatment with an angiogenesis inhibitor, such as for example bevacizumab and vanucizumab, said method comprising detecting frequency of mismatch repair (MMR) deficiency as a biomarker for predicting the patient's response to treatment.

## Description

### BACKGROUND OF THE INVENTION

Anti-angiogenic therapies for cancer were initially conceived as a strategy to cut off the blood supply of growing tumors, thus depriving them of oxygen and other essential nutrients¹. As a result of more than a decade of clinical evaluation of anti-angiogenics, it is now apparent that tumor microenvironment and perfusion biology is more complex than initially appreciated, with evidence for a paradoxical benefit of enhanced tumor perfusion induced by inhibition of pro-angiogenic pathways². Thus, beneficial effects of anti-angiogenic therapies such as bevacizumab and vanucizumab, which block the pro-angiogenic factors vascular endothelial growth factor A (VEGF-A), and VEGF-A along with angiopoietin-2 (Ang-2), respectively, are commonly attributed to normalization of tumor neovasculature, which relieves pro-tumorigenic and immune suppressive effects of hypoxia. VEGF inhibition with bevacizumab and other agents has provided broad-based but incremental benefit, which could be partly due to lack of biomarkers with which to select patients most likely to respond to these therapies².

Immunohistochemical analysis of certain DNA repair proteins has been disclosed as potential biomarker for stratification of patients who receive cisplatin based therapy in specific types of non small cell lung cancer (W.E. Pierceall et al, Ann Oncology, 2012). It has now surprisingly been found that certain DNA mismatch repair genes (herein "MMR genes") serve as biomarker for prediction of cancer patient's benefit to treatment with angiogenesis inhibitors.

### SUMMARY OF THE INVENTION

In one embodiment, the present invention provides a method to predict a cancer patient's response to treatment, comprising detecting the activity or expression level of said patient's DNA mismatch repair (MMR) genes in a sample obtained from that patient, comparing that activity to a reference level or set of reference levels, and using said comparison as a biomarker for predicting said patient response to treatment with a medicament, wherein the medicament comprises a compound acting as inhibitor of angiogenesis.

In another embodiment, the present invention provides a method to predict a cancer patient's response to treatment with an anti-angiogenic therapy, comprising calculating a value representig the activity of said patients DNA mismatch repair (MMR) genes, detected in a sample obtained from that patient, comparing that data to a reference level or set of reference levels, and determining whether said patient benefits from anti-angiogenesis therapy.

In another aspect the present invention relates to a method for predicting the response of a patient having cancer to therapy, wherein the patients therapy comprises the treatment with a compound which acts as angiogenesis inhibitor, said method comprising the step of calculating the frequency of DNA MMR deficiency in a sample obtained from said patient, and treating the patient with a medicament comprising a compound acting as angiogenesis inhibitor, when said value is above a reference level.

: In another aspect the present invention relates to a method for predicting the response of a patient having cancer to therapy, wherein the patients therapy comprises the treatment with a compound which acts as angiogenesis inhibitor, said method comprising the steps of
a) measuring the defects within one or several MMR genes, selected from the group consisting of MSH2, MSH3, MSH6, MLH 1, MLH3, PMS1 and PMS2 in a sample obtained from the cancer patient;
b) calculating a value or values representing the frequency of MMR deficiency from the data obtained in a); and
c) comparing the value or values obtained from step a) and b) to a standard value or set of standard (or reference) values; and
d) treating the patient with a medicament comprising a compound acting as angiogenesis inhibitor, when said value is above a reference level.

In another aspect, the present invention relates to a method for predicting the response of a patient having cancer to therapy, wherein the patient's therapy comprises the treatment with a compound which acts as VEGF- or Ang-2 inhibitor, such as for example the molecules with the INN's bevacizumab and vanucizumab.

In a further aspect, the invention relates to a method of treating a neoplastic disease, such as cancer, in a patient in need thereof, comprising measuring the frequency of MMR deficiency in a sample obtained from the patient to detect a value or values representing that frequency, and treating the patient with a medicament comprising a compound acting as angiogenesis inhibitor, when said value is above a reference level.

In yet another aspect, the invention relates to a kit for predicting the response of a cancer patient's treatment with a medicament comprising an angiogenesis inhibitor, as defined herein, comprising reagents necessary for measuring the frequency of MMR deficiency in a sample obtained from said patient. The kit also comprises a comparator module which comprises a standard value or set of standard values to which the level of response in the sample is compared, together with instructions how to make such comparison. The kit may also comprise one or more other therapeutically active agent(s) for combination with the angiogenesis inhibitor as defined herein.

More preferably the kit comprises a compound acting as angiogenesis inhibitor, preferably as inhibitors of VEGF or Ang-2, such as for example the molecules with the INN's bevacizumab and vanucizumab.

As such, the present invention relates to a method for identifying sensitivity to anti-angiogenesis therapy. Furthermore, the present invention relates to a method for treating a cancer patient with a VEGF inhibitor, such as for example bevacizumab, or an Ang-2 inhibitor such as vanucizumab, by testing the patient for the relative amount (frequency) of one or several deactivated MMR genes selected from the group consisting of MSH2, MSH3, MSH6, MLH1, MLH3, PMS1 and PMS2.

The present invention also relates to the use of MMR deficiency as defined herein, in particular the frequency of MMR deficiency (in %) of one or several MMR genes independently selected from MSH2, MSH3, MSH6, MLH1, MLH3, PMS1 and PMS2, as a biomarker to predict or determine the response of a patient suffering from cancer to treatment with a VEGF- or Ang-2 inhibitor as defined herein.

### DETAILED DESCRIPTION OF THE INVENTION

### Inhibitory effects of VEGF upon adaptive immunity

In addition to their roles in promoting vessel proliferation³, pro-angiogenic factors such as VEGF and Ang-2 also exert suppressive effects upon tumor-infiltrating immune cells - there is substantial overlap between cell types and factors involved in immune suppression in the tumor microenvironment and the regulation of neovascularization⁴. VEGF-A, which is produced by endothelial cells, tumors, and tumor-associated myeloid cells, suppresses anti-tumor immune responses via several mechanisms, including inhibition of dendritic cell maturation⁵, induction of Fas ligand expression on tumor endothelium⁶, and promotion of expression of inhibitory immune checkpoint molecules on intratumoral VEGF-R2+ CD8 + T cells⁷. Thus, in addition to anti-angiogenic and vascular normalization effects, VEGF blockade is also likely to relieve inhibition of intra-tumoral T cells.

### Ang-2 and immune suppression in the tumor microenvironment

As the main ligand of the Tie-2 receptor in the tumor microenvironment, Ang-2 promotes angiogenesis together with VEGF-A³. Ang-2 mediates experimental resistance to VEGF-A inhibition in the context of anti-tumor therapy⁸, and compensatory tumor revascularization and growth during anti-VEGF therapy in renal cell carcinoma⁹. Indeed, simultaneous neutralization of Ang-2 and VEGF with bi-specific antibody retards tumor growth in several xenograft mouse models, with enhanced effects seen upon larger tumors¹⁰.

In addition to direct effects on tumor endothelium, ligation of mouse and human Tie-2-expressing monocytes/macrophages by Ang-2 drives their pro-angiogenic phenotype in tumors^{11,12} and also promotes T cell-mediated immune suppression¹³. Thus, inhibition of Ang-2 is likely to mediate anti-tumor effects via direct effects on tumor vasculature, antagonism of the angiogenic phenotype of tumor-associated macrophages, and relieving inhibition of anti-tumor T cell responses.

### Anti-angiogenesis and immunotherapy

There exists both preclinical and clinical evidence for a beneficial effect of anti-angiogenesis in the context of immunotherapy. VEGF blockade improves tumor accumulation of antigen-specific T cells in a murine melanoma model¹⁴. Although this was mainly interpreted as an effect of normalized vessels that allowed better infiltration, another preclinical study showed that VEGF inhibition enhanced the anti-tumor activity of agonistic CD40 antibody, with enhanced anti-tumor effector T cell function¹⁵. Finally, promising results from a clinical study combining ipilimumab and bevacizumab were recently reported, including increased T cell frequency in post-treatment biopsies, and increased frequency in circulating memory T cells¹⁶.

### A genomic basis for responses to immune checkpoint blockade in melanoma and colorectal cancer

Whole exome sequencing of tumors from melanoma patients has shown a clear correlation between response to CTLA-4 blockade and presence of immunogenic neoepitopes recognized by T cells¹⁷, suggesting that a high mutational load in tumors is the basis for particularly remarkable responses to checkpoint inhibitors. This is consistent with the correlation seen between clinical responses to PD-1 blockade and accumulation of specific T cell clones that pre-exist in tumors prior to efficacious therapy¹⁸. Furthermore, the tumor of a colorectal cancer patient with exceptional response to PD-1 blockade was found to be deficient in DNA mismatch repair (MMR)¹⁹, which results in very high tumor mutation rates. This finding prompted a randomized phase II trial in which responses to PD-1 blockade were compared in patients with MMR-proficient and -deficient colorectal tumors, with responses highly correlated with MMR deficiency and thus high mutational burden²⁰.

Therefore, in one embodiment, the present invention provides a method to predict a cancer patient's response to treatment, comprising detecting the activity of said patients DNA mismatch repair (MMR) genes in a sample obtained from that patient, for example prior to treatment (at baseline), comparing that activity to a reference level or set of reference levels, and using said comparison as a biomarker for predicting said patient response to treatment with a medicament, wherein the medicament comprises a compound acting as inhibitor of angiogenesis.

In another embodiment, the present invention provides a method to predict a cancer patient's response to treatment with an anti-angiogenic therapy, characterized in calculating a value representig the activity of said patients DNA mismatch repair (MMR) genes, detected in a sample obtained from that patient, comparing that data to a reference level or set of reference levels, and determining whether said patient benefits from anti-angiogenesis therapy.

In a further embodiment the present invention relates to a method to predict the response of a patient having cancer to therapy, wherein the patients therapy comprises the treatment with a compound which acts as angiogenesis inhibitor, said method comprising the step of calculating the frequency of DNA MMR deficiency in a sample obtained from said patient prior to treatment, and treating the patient with a medicament comprising a compound acting as angiogenesis inhibitor, when said value is above a reference level.

In one embodiment, the methods as defined herein before are *in vitro* methods.

The term "anti-angiogenic therapy" as used herein means the treatment of a neoplastic disease, such as cancer, by slowing or inhibiting the formation and/or growth of new blood vessels needed for tumor proliferation.

The term "angiogenesis-inhibitor" (or inhibitor of angiogenesis), as used herein, is any compound suitable for use in an anti-angiogenic therapy as defined herein. In one embodiment, an angiogenesis-inhibitor is a VEGF-inhibitor, more specifically a compound inhibiting vascular endothelial growth factor A (VEGF-A). In another embodiment, an angiogenesis inhibitor is an inhibitor of angiopoietin-2 (Ang-2). In a further embodiment, the VEGF- or VEGF-A-inhibitor is a recombinant humanized monoclonal antibody. In another, more specific embodiment said VEGF- or VEGF-A-inhibitor is the molecule with the INN bevacizumab. This molecule is marketed under the tradename Avastin™ by F. Hoffmann-La Roche Ltd. In accordance with the present invention, the VEGF-inhibitor can be administered alone or in combination with additional chemotherapeutic agents. In one embodiment, the VEGF-inhibitor can be combined with an inhibitor of Ang-2. The combination can be carried out sequentially or simultaneously. A simultaneous administration can for example be achieved with two distinct active ingredients against each target (i.e. VEGF and Ang2), or by one molecule with bispcific functionality, such as e.g. a bispecific antibody. Therefore, in another embodiment, the angiogenesis inhibitor in accordance with the present invention is the molecule with the INN vanucizumab. In yet another embodiment the anti-angiogenic therapy according to the present invention can involve the addition of yet another component selected from cancer immunotherapy of chemotherapy. In a specific embodiment the additional component is an agonistic CD40 antibody. Within this embodiment, said agonistic CD40 antibody comprises the heavy chain - and light chain variable domain amino acid sequences of antibody 21.4.1 (ATCC Deposit No. PTA-3605). In yet another embodiment, said CD40 antibody consists of the heavy chain - and light chain amino acid sequences of antibody 21.4.1 (ATCC Deposit No. PTA-3605). Information for the administration (does, dosage schedule or administration route) of the specific angiogenesis inhibitors and CD40 agonist mentioned herein are readily available to the skilled person, such as a clinical oncologist.

The term "response" as used herein means meeting any clinical endpoint. Preferably the clinical endpoint is beneficial for the patient, when for example compared to placebo or standard of care. In one embodiment clinical endpoint, or response, as used herein means Overall Survival (OS), Progression Free Survival (PFS) or Event Free Survival (EFS). In another embodiment the term response refers to Response Evaluation Criteria in Solid Tumors (RECIST) designations, such as complete response (CR), partial response (PR), or stable disease (SD).

The term "MMR genes" means one or several genes independently selected from the group consisting of MSH2, MSH3, MSH6, MLH1, MLH3, PMS1, PMS2, XPF, BRCA1, ERCC1, PARP1, ATM and p53. In one embodiment, the term "MMR genes" means one or several MMR genes independently selected from the group consisting of MSH2, MSH3, MSH6, MLH1, MLH3, PMS1 and PMS2. In another embodiment, combinational subsets of said MMR genes can also be used. Within this embodiment, preferred subsets comprise any 2, or 3, or 4, or 5, or 6 or all of the MMR genes as indicated above.

The "activity" of mismatch repair (MMR) genes according to the present invention means that these genes exercise their function of repairing mutated or otherwise not correctly replicated DNA. Any loss of MMR activity may be due to defects in the MMR genes, for example due to mutations or lesions, and may result in a lower expression level of the corresponding gene products, such as for example proteins involved in DNA damage response. Therefore, in one embodiment of the present invention, the term "frequency of MMR deficiency" means a value or values representing the relative amount of one or several MMR genes which have lost some expression or activity.

The frequency of MMR deficiency obtained from a patient's sample in accordance with the present invention is compared to a standard or reference value or set of standard values. A "standard (or reference) value" can be any suitable value to distinguish responders from non-responders. In one embodiment the standard value is a threshold value, or set of values, for the frequency of defects in MMR genes according to the present invention, whereby a frequency above said threshold value, or set of values, indicates the patient's likelihood to respond to, and benefit from, treatment with an angiogenesis inhibitor. In another embodiment, the standard value is obtained from a patient suffering from the same type of cancer. In a further embodiment, a frequency of MMR deficiency above about 10%, or above about 15%; or above about 20%; or above about 25%; or above about 30%; or above about 35%; or above about 40%; or above about 50% indicates the patient's likelihood to respond to, and benefit from, treatment with an angiogenesis inhibitor.

In another embodiment, the standard value is a cut-off point, or cut-off points, for the expression level of said MMR genes, or subsets, as defined herein. Within this embodiment the expression level for said MMR gene, or subsets of genes, is compared to the expression level of the same MMR gene, or subsets, detected for example in a sample obtained from a patient suffering from the same type of cancer. Patient stratification in this embodiment is made by referring to a higher or lower MMR gene expression level (profile), wherein a lower expression level indicates that a patient is more likely to benefit from treatment with an angiogenesis inhibitor as defined herein.

In yet another embodiment, the frequency of MMR deficiency of one or more MMR genes as defeined herein is combined with immunohistochemical analysis of said MMR gene's protein expression and/or whole exome sequencing to assess global tumor mutation rates. Algorithms which combine these parameters may provide a value, or set of values, which provide a more accurate prediction of a patient's response to treatment with an angiogenesis inhibitor, and thus form a further embodiment of the present invention.

The defects within the MMR genes in accordance with the present invention and/or corresponding expression levels can be detected by any technique known to the person of skill in the art for analysis or amplification of genes and/or gene expression profiles. In one embodiment, expression levels are detected using immunohistochemical methods followed by image acquisition and statistical analysis, well known to the person of skill in the art.

The term "cancer" (or "neoplastic disease") as used herein means breast cancer, prostate cancer, renal cancer, lung cancer, colorectal cancer (i.e. including colon cancer and rectal cancer), ovarian cancer, carcinoid, NHL, cervical cancer, glioblastoma, gastric cancer or pancreatic cancer. In one, more specific embodiment the cancer is colorectal cancer (i.e. including colon cancer and rectal cancer) and ovarian cancer.

The "sample" obtained from a patient in accordance with the present invention is any sample suitable for detecting or analyzing said MMR genes. In one embodiment, said sample is a blood sample or a tumor biopsy sample. The sample can be obtained from said patient at any time during treatment. In one embodiment, the sample is obtained prior to treatment (at baseline) with said angiogenesis inhibitor.

Thus, in one embodiment, the present invention provides a method for predicting, a cancer patient's response to treatment with an angiogenesis inhibitor, characterized in that the higher the frequency of MMR deficiency of one or several MMR genes, or the lower the expression level of said MMR genes, detected in a sample obtained from that patient, the higher is the likelihood of said patient's response to said treatment as for example indicated by ROC (AUC) values and analyses, well known to the person of skill in the art of clinical pharmacology. Within this embodiment, the MMR genes are independently selected from MSH2, MSH3, MSH6, MLH 1, MLH3, PMS1 and PMS2.

In another embodiment, the present invention provides a method for determining a cancer patient's response to treatment with an angiogenesis inhibitor, comprising the following steps:
a) taking a sample from the patient;
b) measuring the expression level of one or several MMR genes in the sample;
c) calculating a value representing said expression level measured in b);
d) comparing said value calculated in c) from the patient to standard values (expression levels), for example from a patient having the same type of cancer; and
e) administering a compound which acts as inhibitor of angiogenesis, when the value calculated in c) is below said standard value.
In one, more specific embodiment, the method is an *in vitro* method; the sample in a) is obtained prior to start of treatment; and the MMR genes in b) are selected from one or several of the group consisting of MSH2, MSH3, MSH6, MLH1, MLH3, PMS1 and PMS2; and the angiogenesis inhibitor in e) is selected from bevacizumab and/or vanucizumab.

In another embodiment, the present invention relates to a method for predicting the response of a patient having cancer to therapy, wherein the patients therapy comprises the treatment with a compound which acts as angiogenesis inhibitor, said method comprising the steps of
a) measuring defects within one or several MMR genes, selected from the group consisting of MSH2, MSH3, MSH6, MLH1, MLH3, PMS1 and PMS2 in a sample pre-obtained from the cancer patient;
b) calculating a value or values representing the frequency of MMR deficiency; and
c) comparing the value or values obtained from step a) and b) to a standard value or set of standard (or reference) values; and
d) treating the patient with a medicament comprising a compound acting as angiogenesis inhibitor, when said value is above a reference level.

In another embodiment, there is provided an *in vitro* method of identifying a patient suffering from a neoplastic disease, such as e.g. cancer, as likely to respond to a therapy comprising an angiogenesis inhibitor, the method comprising,
a) measuring defects within one or several MMR genes in a sample obtained from that patient prior to treatment;
b) calculating a frequency of MMR deficieny from the data obtained in a);
c) comparing said frequency from b) to a reference level; and
d) identifying said patient as more likely to respond to the therapy comprising said angiogenesis inhibitor when the frequency obtained in b) is above said reference level, and wherein
the MMR genes in a) are selected from one or several of the group consisting of MSH2, MSH3, MSH6, MLH 1, MLH3, PMS1 and PMS2.

Within these embodiments, the frequency obtained in b) above the reference level indicates a patient's high likelihood to respond to treatment with an angiogenesis inhibitor, whereas a relative amount below said level indicates that said patient is less likely to respond to that treatment.

### Method of Treatment

In yet another embodiment, the invention also involves a method of treatment, wherein the activity of a patients MMR genes, preferably the expression level or profile, is first detected from a sample obtained from a patient suffering from cancer for sensitivity relative to a standard level or set of standard levels or pre-treatment initiation levels and then an angiogenesis-inhibitor is administered. The angiogenesis inhibitor is as defined herein. Preferably said angiogenesis-inhibitor is bevacizumab or vanucizumab. Said compounds may be administered according to pharmaceutical preparations, dosages and dosage regimen well known to a person skilled in the art. More particularly, compositions for intravenous (iv) or subcutaneous (sc) administration are preferred.

Within this embodiment, bevacizumab and vanucizumab can be administered alone or in combination with one or more other therapeutic agents. Possible combination therapy may take the form of fixed combinations, or the administration of a compound of the invention and one or more other therapeutic agents which are staggered or given independently of one another, or the combined administration of fixed combinations and one or more other therapeutic agents.

Also within this embodiment, bevacizumab and vanucizumab can, besides or in addition, be administered especially for tumour therapy in combination with chemotherapy (cytotoxic therapy), targeted therapy, endocrine therapy, radiotherapy, immunotherapy, surgical intervention, or a combination of these. Long-term therapy is equally possible as is adjuvant therapy in the context of other treatment strategies, as described above. Other possible treatments are therapy to maintain the patient's status after tumour regression, or even chemo-preventive therapy for example in patients at risk. In one embodiment, bevacizumab and/or vanucizumab are combined with immunotherapy, preferably with an agonistic CD40 antibody. Within this embodiment, said agonistic CD40 antibody comprises the heavy chain - and light chain variable domain amino acid sequences of antibody 21.4.1 (ATCC Deposit No. PTA-3605). In yet another embodiment, said CD40 antibody consists of the heavy chain - and light chain amino acid sequences of antibody 21.4.1 (ATCC Deposit No. PTA-3605).

### Kit and Device

In one aspect the invention relates to a kit and in another aspect to a device for predicting the response, preferably of a cancer in a subject (or patient), to an angiogenesis inhibitor as defined herein, preferably bevacizumab and/or vanucizumab, said kit comprising means, tools or devices for sample collection, especially for collection of blood- or tumor biopsy samples, and instructions how to detect the activity of MMR genes as defined herein, especially expression levels or profiles of one or several genes independently selected from MSH2, MSH3, MSH6, MLH 1, MLH3, PMS1 and PMS2 in said samples.

The kit and device may also preferably comprise a comparator module which comprises a standard (or reference) value or set of standard values to which the activity of said MMR genes, or the frequency of MMR deficiency, or the expression level as calculated in accordance with the present invention, is compared.

In another embodiment, there is provided a kit for predicting the response to treatment with a medicament comprising a compound which acts as inhibitor of angiogenesis, comprising;
a) means for obtaining a sample, preferably a blood- or tumor biopsy sample, from a patient suffering from cancer;
b) instructions how to detect the activity of MMR genes from that sample;
c) a comparator module, comprising standard (or reference) values and instructions how to use them; and
d) a medicament comprising a compound which acts as inhibitor of angiogenesis.
Within this embodiment, the activity of MMR genes in b) is preferably represented by the relative amount of defects in or the expression level of one or several MMR genes as defined herein. In a more specific embodiment, the MMR genes are selected from MSH2, MSH3, MSH6, MLH1, MLH3, PMS1 and PMS2. In another embodiment, the medicament in d) is preferably bevacizumab or vanucizumab.

In still another embodiment, there is provided the use of MMR deficiency as defined herein, in particular the frequency of MMR deficiency or the expression level of one or several MMR genes as defined herein, as a biomarker to predict a patients response to treatment with a VEGF- or Ang-2 inhibitor as defined herein. In a more specific embodiment, the patient referred to herein is suffering from cancer, more particularly a solid tumor, especially ovarian or colorectal cancer, and the one or several MMR genes are independently selected from MSH2, MSH3, MSH6, MLH1, MLH3, PMS1 and PMS2.

The following examples are illustrative of the invention and not limitative thereof.

### EXAMPLES

### EXAMPLE 1

### Response rates to single agent bevacizumab are consistent with expected rates of MMR deficiency (dMMR) in some tumor types

Literature research and internal data (F. Hoffmann-La Roche AG, Basel, Switzerland) demonstrates that response rates to single agent bevacizumab are consistent with expected rates of MMR deficiency in ovarian cancer and colorectal cancer:

| | Response Rate* to Single Agent Bevacizumab | Frequency of Mismatch Repair Deficiency |
|---|---|---|
| Ovarian cancer | 17.7% (n=62) | 2-29%²¹ |
| Colorectal cancer | 3.0% (n=232) | 3.5%²², 5.0%²³ |

| | | |
|---|---|---|
| *internal data from F. Hoffmann-La Roche AG metaanalysis, n=1686 ²¹n=515 ²³n=153 | | |

References cited herein
1 Folkman, J. Tumor angiogenesis: therapeutic implications. N Engl J Med 285, 1182-1186, doi:10.1056/NEJM197111182852108 (1971).
2 Jain, R. K. Antiangiogenesis strategies revisited: from starving tumors to alleviating hypoxia. Cancer Cell 26, 605-622, doi:10.1016/j.ccell.2014.10.006 (2014).
3 Eklund, L. & Saharinen, P. Angiopoietin signaling in the vasculature. Exp Cell Res 319, 1271-1280, doi:10.1016/j.yexcr.2013.03.011 (2013).
4 Rivera, L. B. & Bergers, G. Intertwined regulation of angiogenesis and immunity by myeloid cells. Trends Immunol 36, 240-249, doi:10.1016/j.it.2015.02.005 (2015).
5 Gabrilovich, D. I. et al. Production of vascular endothelial growth factor by human tumors inhibits the functional maturation of dendritic cells. Nat Med 2, 1096-1103 (1996).
6 Motz, G. T. et al. Tumor endothelium FasL establishes a selective immune barrier promoting tolerance in tumors. Nat Med 20, 607-615, doi:10.1038/nm.3541 (2014).
7 Voron, T. et al. VEGF-A modulates expression of inhibitory checkpoints on CD8+ T cells in tumors. J Exp Med 212, 139-148, doi:10.1084/jem.20140559 (2015).
8 Rigamonti, N. et al. Role of angiopoietin-2 in adaptive tumor resistance to VEGF signaling blockade. Cell Rep 8, 696-706, doi:10.1016/j.celrep.2014.06.059 (2014).
9 Wang, X. et al. The role of angiopoietins as potential therapeutic targets in renal cell carcinoma. Transl Oncol 7, 188-195, doi:10.1016/j.tranon.2014.02.003 (2014).
10 Kienast, Y. et al. Ang-2-VEGF-A CrossMab, a novel bispecific human IgG1 antibody blocking VEGF-A and Ang-2 functions simultaneously, mediates potent antitumor, antiangiogenic, and antimetastatic efficacy. Clin Cancer Res 19, 6730-6740, doi:10.1158/1078-0432.CCR-13-0081 (2013).
11 De Palma, M. et al. Tie2 identifies a hematopoietic lineage of proangiogenic monocytes required for tumor vessel formation and a mesenchymal population of pericyte progenitors. Cancer Cell 8, 211-226, doi:10.1016/j.ccr.2005.08.002 (2005).
12 Guex, N. et al. Angiogenic activity of breast cancer patients' monocytes reverted by combined use of systems modeling and experimental approaches. PLoS Comput Biol 11, e1004050, doi:10.1371/journal.pcbi.1004050 (2015).
13 Coffelt, S. B. et al. Angiopoietin 2 stimulates TIE2-expressing monocytes to suppress T cell activation and to promote regulatory T cell expansion. J Immunol 186, 4183-4190, doi:10.4049/jimmunol.1002802 (2011).
14 Shrimali, R. K. et al. Antiangiogenic agents can increase lymphocyte infiltration into tumor and enhance the effectiveness of adoptive immunotherapy of cancer. Cancer Res 70, 6171-6180, doi:10.1158/0008-5472.CAN-10-0153 (2010).
15 Selvaraj, S., Raundhal, M., Patidar, A. & Saha, B. Anti-VEGF antibody enhances the antitumor effect of CD40. Int J Cancer 135, 1983-1988, doi:10.1002/ijc.28833 (2014).
16 Hodi, F. S. et al. Bevacizumab plus ipilimumab in patients with metastatic melanoma. Cancer Immunol Res 2, 632-642, doi:10.1158/2326-6066.CIR-14-0053 (2014).
17 Snyder, A. et al. Genetic basis for clinical response to CTLA-4 blockade in melanoma. N Engl J Med 371, 2189-2199, doi:10.1056/NEJMoa1406498 (2014).
18 Tumeh, P. C. et al. PD-1 blockade induces responses by inhibiting adaptive immune resistance. Nature 515, 568-571, doi:10.1038/nature13954 (2014).
19 Lipson, E. J. et al. Durable cancer regression off-treatment and effective reinduction therapy with an anti-PD-1 antibody. Clin Cancer Res 19, 462-468, doi:10.1158/1078-0432.CCR-12-2625 (2013).
20 Le, D. T. et al. PD-1 Blockade in Tumors with Mismatch-Repair Deficiency. N Engl J Med 372, 2509-2520, doi:10.1056/NEJMoa1500596 (2015).
21 Xiao, X., Melton, D. W. & Gourley, C. Mismatch repair deficiency in ovarian cancer -- molecular characteristics and clinical implications. Gynecol Oricol 132, 506-512, doi:10.1016/j.ygyno.2013.12.003 (2014).
22 Koopman, M. et al. Deficient mismatch repair system in patients with sporadic advanced colorectal cancer. Br J Cancer 100, 266-273, doi:10.1038/sj.bjc.6604867 (2009).
23 Venderbosch, S. et al. Mismatch repair status and BRAF mutation status in metastatic colorectal cancer patients: a pooled analysis of the CAIRO, CAIRO2, COIN, and FOCUS studies. Clin Cancer Res 20, 5322-5330, doi:10.1158/1078-0432.CCR-14-0332 (2014).

## Claims

1. An *in vitro* method to predict a cancer patient's response to treatment, comprising detecting the activity of said patients DNA mismatch repair (MMR) genes in a sample obtained from that patient, comparing that activity to a reference level or set of reference levels, and using said comparison as a biomarker for predicting said patient response to treatment with a medicament, wherein the medicament comprises a compound acting as inhibitor of angiogenesis.

2. The method according to claim 1 comprising the following steps:
a) taking a sample from the patient;
b) measuring the expression level of one or several MMR genes in the sample;
c) calculating a value representing said expression level measured in b);
d) comparing said value calculated in c) from the patient to standard values (expression levels), for example from a patient having the same type of cancer; and
e) administering a compound which acts as inhibitor of angiogenesis, when the value calculated in c) is below said standard value.

3. The method according to claim 2, wherein the sample in a) is obtained prior to start of treatment; and the MMR genes in b) are selected from one or several of the group consisting of MSH2, MSH3, MSH6, MLH1, MLH3, PMS1 and PMS2.

4. The method according to claim 1, said method comprising the steps of
a) measuring defects within one or several MMR genes, selected from the group consisting of MSH2, MSH3, MSH6, MLH1, MLH3, PMS1 and PMS2 in a sample pre-obtained from the cancer patient;
b) calculating a value or values representing the frequency of MMR deficiency; and
c) comparing the value or values obtained from step a) and b) to a standard value or set of standard (or reference) values; and
d) treating the patient with a medicament comprising a compound acting as angiogenesis inhibitor, when said value is above a reference level.

5. The method according to any one of claims 1 to 5, to predict a cancer patient's response to treatment with bevacizumab and/or vanucizumab, wherein the cancer is a solid tumor, preferably colorectal cancer or ovarian cancer,

6. The use of one or several MMR genes as a biomarker to predict a patients response to treatment with an angiogenesis inhibitor, preferably bevacizumab and/or vanucizumab, wherein said MMR genes are independently selected from MSH2, MSH3, MSH6, MLH1, MLH3, PMS1 and PMS2.
